# EUROPEAN PATENT SPECIFICATION

(11) **EP 1 694 218 B1**
(45) Date of publication and mention of the grant of the patent: **23.02.2011**
(21) Application number: 04813273.2
(22) Date of filing: 07.12.2004
(51) Int. Cl.: A61B 17/122

(54) **LEFT ATRIAL APPENDAGE EXCLUSION DEVICE**
AUSSCHLUSSVORRICHTUNG FÜR DAS LINKE HERZOHR
DISPOSITIF D'EXCLUSION D'APPENDICE DE L'OREILLETTE GAUCHE

(30) Priority: 17.12.2003 US 738017
(43) Date of publication of application: 30.08.2006
(73) Proprietor: Edwards Lifesciences Corporation, Irvine, CA 92614 (US)
(72) Inventor: WILLIAMS, Matthew, New York, NY 10033 (US); WARD, Jim, L., Laguna Niguel, California 92670 (US)
(74) Representative: Parry, Christopher Stephen
(86) International application number: PCT/US2004/040933
(87) International publication number: WO 2005/060838

(56) References cited:
- WO-A-01/97696
- WO-A-03/096881
- US-A- 5 100 416

## Description

### Field of the Invention

The present invention relates generally to medical devices, specifically to a device for excluding the left atrial appendage (LAA) from blood flow in the left atrium, and related procedures.

### Background of the Invention

Embolic stroke is the nation's third leading killer for adults, and is a major cause of disability. There are over 80,000 strokes per year in the United States alone. The most common cause of embolic stroke emanating from the heart is thrombus formation due to atrial fibrillation. Atrial fibrillation is an arrhythmia of the heart that results in a rapid and chaotic heartbeat that produces lower cardiac output and irregular and turbulent blood flow in the vascular system. There are over five million people worldwide with atrial fibrillation, with about four hundred thousand new cases reported each year. Atrial fibrillation is associated with a 500 percent greater risk of stroke due to the condition. A patient with atrial fibrillation typically has a significantly decreased quality of life due, in large part, to the fear of a stroke, and the pharmaceutical regimen necessary to reduce that risk.

For patients who have atrial fibrillation and develop atrial thrombus therefrom, the clot normally occurs in the left atrial appendage (LAA) of the heart. The LAA is a cavity which looks like a small finger or windsock and which is connected to the lateral wall of the left atrium between the mitral valve and the root of the left pulmonary vein. The LAA normally contracts with the rest of the left atrium during a normal heart cycle, thus keeping blood from becoming stagnant therein, but like the rest of the atrium does not contract in patients experiencing atrial fibrillation due to the discoordinate electrical signals associated with AF. As a result, thrombus formation is predisposed to form in the stagnant blood within the LAA. Blackshear and Odell have reported that of the 1288 patients with non-rheumatic atrial fibrillation involved in their study, 221 (17%) had thrombus detected in the left atrium of the heart. Blackshear J.L., Odell, J.A., Appendage obliteration to reduce stroke in cardiac surgical patients with atrial fibrillation, Ann Thorac. Surg., 1996,61(2):755-9. Of the patients with atrial thrombus, 201 (91 %) had the atrial thrombus located within the LAA. The foregoing suggests that the elimination or containment of thrombus formed within the LAA of patients with atrial fibrillation would significantly reduce the incidence of stroke in those patients.

Pharmacological therapies for stroke prevention such as oral or systemic administration of blood thinning agents, such as warfarin, coumadin or the like have been inadequate due to serious side effects of the medications (e.g., an increased risk of bleeding) and lack of patient compliance in taking the medication.

As an alternative to drug therapy, invasive surgical procedures for closing the LAA have been proposed. Most commonly, the LAA has been closed or removed in open surgical procedures, typically where the heart has stopped and the chest opened through the sternum. The perceived risks of even a thorascopic surgical procedure often outweigh the potential benefits, and many patients are not suitable candidates for such surgical procedures due to a compromised condition or having previously undergone cardiac surgery. See Lindsay, B.D., Obliteration of the left atrial appendage: A concept worth testing. Ann Thorac. Surg., 1996.61(2):515.

Because of the significant risk and trauma of such procedures, LAA removal occurs almost exclusively when the patient's chest is opened for other procedures, such as coronary artery bypass or valve surgery. For that reason, alternative procedures that do not require opening of the patient's chest, i.e., a large median sternotomy, have been proposed.

For instance, U.S. Pat. No. 5,865,791, to Whayne et al. describes a transvascular approach for closing the LAA. Access is gained via the venous system, typically through a femoral vein, a right internal jugular vein, or a subclavian vein, where a catheter is advanced in an antegrade direction to the right atrium. The intra-atrial septum is then penetrated, and the catheter passed into the left atrium. The catheter is then positioned in the vicinity of the LAA which is then fused closed, e.g., using radiofrequency energy, other electrical energy, thermal energy, surgical adhesives, or the like. The transvascular approach suggested by Whayne et al. is advantageous in that it avoids the need to penetrate the patient's chest but suffers from the need to penetrate the intra-atrial septum, may not provide definitive closure, requires entry into the LAA, which may dislodge a clot and requires injury to the endocardial surface, which may promote thrombus formation.

U.S. Pat. No. 5,306,234 to Johnson describes a thoracoscopic procedure where access to the pericardial space over the heart is achieved using a pair of intercostal penetrations (i.e., penetrations between the patients ribs) to establish both visual and surgical access. While such procedures may be performed while the heart remains beating, they still require deflation of the patient's lung and that the patient be placed under full anaesthesia. Furthermore, placement of a chest tube is typically required to reinflate the lung.

U.S. Pat. No. 6,488,689 to Kaplan, et al. discloses another minimally invasive approach to LAA closure where access to the pericardial space overlying the patient's LAA is accomplished through percutaneous penetrations through the patient's skin. Rather than passing through the rib cage, as with the technique disclosed in Johnson, Kaplan, et al. rely on a "sub-xiphoid" approach where the percutaneous penetration is first made beneath the rib cage, preferably between the xiphoid and adjacent costal cartilage. An atrial appendage closure tool is advanced through the penetration, over the epicardial surface (in the pericardial space) to reach a location adjacent to the exterior of the LAA. The closure tool can then be used to close the LAA to prevent the formation of clot and the release of emboli from the atrium. Closure can be effected by positioning a loop of material, such as suture, wire, mesh, tape, or the like, over the appendage and cinch the loop tighter to close the interior of the appendage. Other closure techniques disclosed include suturing, stapling, clipping, fusing, gluing, clamping, riveting, or the like.

Despite these efforts, it would be desirable to provide devices and procedures for closure of the LAA that account for the delicate nature of existing thrombi in the LAA cavity and are less traumatic to the LAA tissue and left atrium.
WO-A-01/97696 discloses an aneurism clip or clasp comprising a pair of elongated spaced apart compression members co-extensive with one another, wherein the members form a closed periphery defining an opening therethrough.
WO-A-03/096881 discloses a device in which a left atrial appendage is clamped between a pair of elongate pins movably mounted on a deployment device. The pins are then engaged with one another using end caps and detached from the deployment device.
US-A-5100416 discloses a surgical clip comprising a pair of elongate spaced apart compression members co-extensive with one another, wherein the members form a closed periphery defining an opening therein when the two ends of the members are brought together.

### Summary of the Invention

The present invention provides a left atrial appendage (LAA) exclusion device as described in claim 1.

### Brief Description of the Drawings

Fig. 1 is an anterior view of a heart illustrating the position of the left atrial appendage (LAA) relative to the remaining structures of the heart;

Figs. 2A-2E are schematic sectional views of a number of steps in the deployment of LAA exclusion devices;

Fig. 3 is a sectional view through the deployed LAA exclusion device as seen along line 3-3 of Fig. 2D;

Fig. 4 is a perspective view of an LAA exclusion device having a generally rectangular outer periphery and four inner protrusions;

Figs. 5-7 are perspective views of several LAA exclusion devices having lenticular outer peripheries and various inner protrusions.

Fig. 8 is a perspective view of an LAA exclusion device having a pair of elongated compression members defining a continuous periphery and featuring a plurality of needles extending inward therefrom;

Fig. 9 is a view of a further LAA exclusion device having a pair of elongated compression members hinged together at one end and having mating structures on an opposite end;

Figs. 10A and 10B are plan and views, respectively, of a still further LAA exclusion device that has a pair of non-linear, elongated compression members defining a continuous periphery and having a plurality of needles extending inward thereform;

Figs. 11A and 11B are schematic plan views of the deployment of the LAA exclusion device of Figs. 10A and 10B.
With reference to the devices described in relation to the accompanying drawings, it is to be understood that only the devices shown in Figs. 2A-2D are in accordance with the invention as claimed. The other devices are included for illustrative purposes.

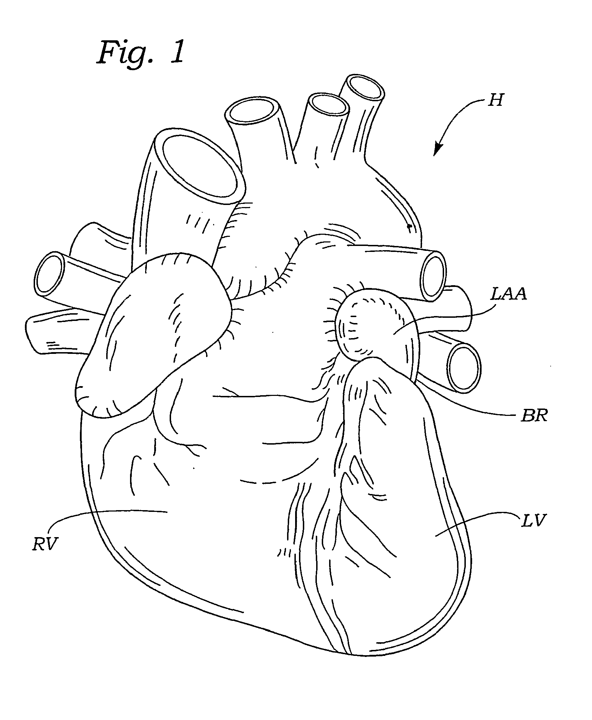

FIG.1 is an anterior view of a heart illustrating the right ventricle RV, the left ventricle LV, and the left atrial appendage LAA. The methods and apparatus described below are intended to place an exclusion device over the base region BR of the left atrial appendage. By closing off the base region BR, the exchange of materials between the left atrial appendage LAA and the left atrium LA will be stopped. Thus, the release of emboli from the left atrial appendage into the left atrium will be stopped.

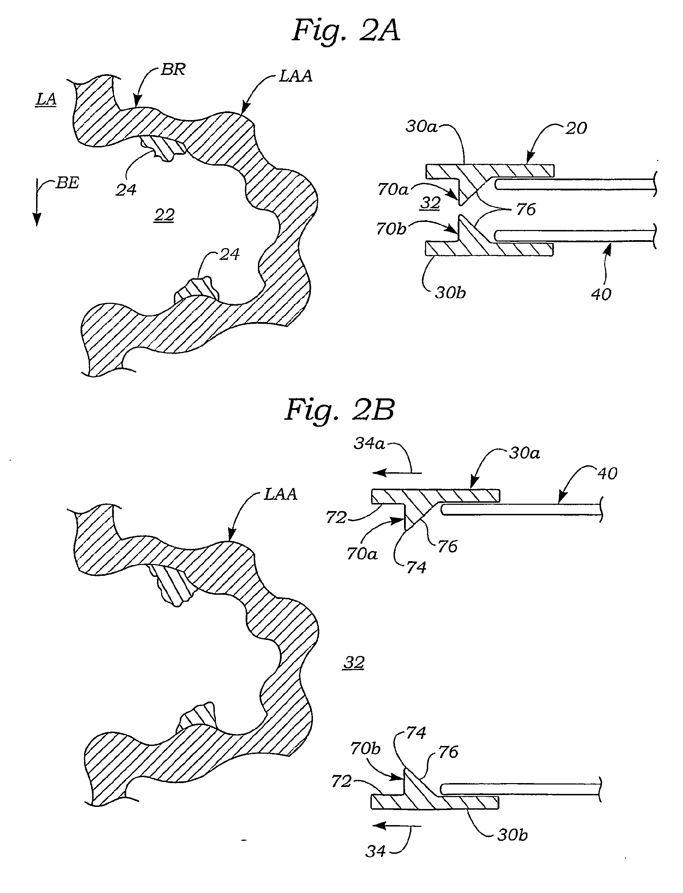

Figs. 2A-2D illustrate several steps in the deployment of an exemplary LAA exclusion device 20 of the present invention to close off the base region BR of a left atrial appendage LAA. A cavity 22 within the left atrial appendage LAA is shown with blood clots or thrombi 24 deposited on the inner wall of the left atrial appendage LAA. As mentioned above, because of the isolated nature of the cavity 22 from the main blood flow within the left atrium LA the thrombus deposits 24 can form and may eventually break free, causing problems with strokes. The design of the exclusion device 20 and its deployment at the base region BR as will be described is intended to prevent extrusion of the thrombus deposits 24 from within the cavity 22 during the procedure.

Fig. 2A shows the exclusion device 20 in a relaxed or closed position. Various embodiments of exclusion devices of the present invention will be described below, and any can be used in the procedure as described. The exclusion device 20 illustrated has a pair of spaced apart compression members 30a, 30b that are biased toward one another. Although not shown, the compression members 30a, 30b are elongated and have opposite ends that can be coupled together. The entire structure can be a single piece of the closed periphery and form the flexible material so that the compression members 30a, 30b are biased toward one another into the closed position of Fig. 2A by their own material elasticity. Alternatively, one end of the two compression members 30a, 30b may be hinged together with the other end closable with a snap or ratchet device or a similar device so as to form a closed periphery.

Whatever the configuration, the compression members 30a, 30b are capable of being expanded or opened into the position shown in Fig. 2B. The compression members 30a, 30b define therebetween an opening 32 that can be enlarged from the minimum size shown in Fig. 2A to the maximum size shown in Fig. 2B. The exclusion device 20 in its closed configuration of Fig. 2A has a relatively small profile and can be introduced into the chest cavity through a plurality of access ports. Once in position adjacent the left atrial appendage LAA, the exclusion device 20 may be expanded as in Fig. 2B so as to be able to surround the left atrial appendage LAA. Movement arrows 34 indicate the advancement of the exclusion device 20 toward and over the left atrial appendage LAA.

It should be noted at this stage that various medical implements are suitable for manipulating the exclusion device 20 within the chest cavity. For example, forceps 40 are shown extending within the opening 32 to contact the inner side of each compression members 30a, 30b. Opening the forceps 40 as seen in Fig. 2B applies an outward to the compression members 30a, 30b from within the opening 32. It will be apparent to one of skill in the art that many different types of implements maybe used to manipulate the exclusion device 20

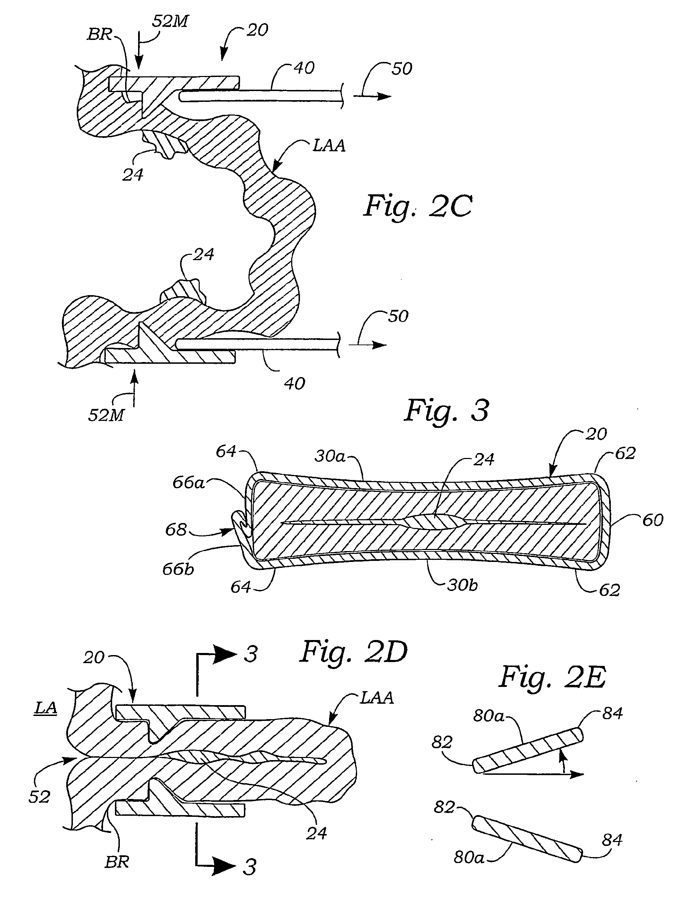

Now with reference to Figure 2C, the exclusion device 20 is shown still in its open configuration after having been advanced to the base region BR of the left atrial appendage LAA. At this stage, movement arrows 50 indicate retraction of the forceps 40. Once retracted, the outward that had been applied to the compression members 30a, 30b is removed and they are permitted to close toward one another, as indicated by arrows 52m. Again, this inward movement can be caused by the natural resiliency of the material of the exclusion device 20, or may be externally applied (e.g., in the case of a hinged exclusion device).

Finally, the exclusion device 20 is shown in Fig. 2D after compressing the left atrial appendage LAA so as to exclude the cavity 22 therein from the left atrium LA. As mentioned above, the exclusion device 20 is desirably advanced and closed at the base region BR of the left atrial appendage LAA so as to exclude the entire cavity 22 (see Fig. 2C) from the left atrium LA. From the left atrium LA, a relatively smooth inner wall 52 is the result, thus minimizing any future thrombus formations.

Fig. 3 is a sectional view taken along line 3-3 of Fig. 2D showing the width of the exclusion device 20 and its generally rectangular cross-section. The left atrial appendage LAA is seen compressed flat within the exclusion device 20, and any thrombus deposits 24 that had resided within the cavity 22 are captured and trapped therein. It should be noted that the generally rectangular cross-section of the exclusion device 20 avoids compressing any portion of the left atrial appendage LAA tissue more than the other portions. That is, the lateral ends of the device 20 are approximately the same dimension as the middle portion. This helps prevent trauma to the LAA tissue by avoiding overly pinching the lateral ends thereof.

The exemplary exclusion device 20 includes the aforementioned compression members 30a, 30b that are relatively elongated (by comparison of the length seen in Fig. 3 vs. the width seen in Fig. 2D). One end 62 of each of the compression members 30a, 30b is joined to the corresponding end 62 of the other compression member at a hinge wall 60. The hinge wall 60 is shown as an integral, contiguous part of the material of the compression members 30a, 30b (the entire structure may be one piece) and thus functions as a living hinge by using relatively flexible or elastic material. Alternatively, the compression members 30a, 30b may be separately formed elements that are joined together using a conventional hinge (not shown) at the ends 62.

The opposite end 64 of each of the compression members 30a, 30b is joined to corresponding end 64 of the other compression member using mating structure that mutually engage to form a closed periphery (generally rectangular as shown) for the exclusion device 20. In the illustrated embodiment, the mating structure includes a pair of walls 66a, 66b each of which terminate in a mating rib or tooth 68. Of course, the mating structures can be any number of configurations including multiple ratchet teeth for a variable sized closed periphery, hook and loop fasteners, etc.

In a preferred embodiment, at least one of the compression members 30a, 30b has a protrusion facing the opening 32 that helps prevent migration of the exclusion device 20 after deployment. In the embodiment of Figs. 2A-3, a protrusion 70a, 70b extends inward toward the opening 32 from both of the compression members 30a, 30b. The presence of the protrusions 70a, 70b helps prevent movement of the deployed exclusion device 20 as seen in Fig. 2D because they press into the exterior tissue of the left atrial appendage LAA and thus provide greater frictional resistance than without the protrusions. In the illustrated embodiment, the compression members 30a, 30b each comprise an elongated strip of material having a planar inner surface 72 (see Fig. 2B) from which the protrusions 70a, 70b extend.

Desirably, the protrusions 70a, 70b are shaped so as to be atraumatic to the exterior tissue of the left atrial appendage LAA. As seen best in Fig. 2B, each protrusion 70a, 70b includes a relatively rounded inner apex 74 forming the inner end of a generally triangular cross-section. This configuration permits the protrusions 70a, 70b to press into the soft tissue of the left atrial appendage LAA without causing short- or long-term damage thereto. Of course, other shapes and configurations of protrusions are contemplated, as will be seen below.

In addition to helping prevent migration of the deployed exclusion device 20, the protrusions 70a, 70b may also help prevent extrusion of any thrombus deposits from within the cavity 22 of the left atrial appendage LAA during deployment. More generally, the present invention contemplates a deployment procedure wherein the left atrial appendage LAA is first squeezed together close to the wall of the left atrium LA and then in a direction away from the atrium; that is, there is a squeezing motion away from the left atrium LA. This can be done in a number of ways, although as seen in Figs. 2A-2B, the protrusions 70a, 70b each include an angled compression surface 76 that each apply a non-planar compressive force to opposite sides of the left atrial appendage LAA during deployment of the exclusion device 20. The angled orientation of the surfaces 76 provide the squeezing motion away from the left atrium LA. The preferred angle of the compression surfaces 76 is shown as about 45 degrees, although smaller angles on correspondingly wider protrusions 70a, 70b are contemplated. As the angle of the compression surfaces 76 increases, the protrusions 70a, 70b become more pointed which may ultimately cause undue trauma to the left atrial appendage LAA tissue.

Instead of providing the ramped protrusions 70a, 70b, alternative compression members 80a, 80b may be oriented at an angle with respect to one another as seen in Fig. 2E, as opposed to being oriented in parallel as shown in Figs. 2A-2D. More specifically, the leading ends 82 of the compression members 80a, 80a that are advanced first and positioned adjacent the exterior wall of the left atrium LAA are spaced closer together than the opposite or trailing ends 84. Each compression member 80a, 80b is oriented an angle θ with respect to a bisecting plane therebetween, which angle is desirably between 5-45 degrees, more preferably between 15-45 degrees. This creates a non-planar compressive force on opposite sides of the left atrial appendage LAA by the opposed ramp surfaces on the exclusion device. Deployment of the alternative compression members 80a, 80b causes the leading ends 82 to contact the exterior tissue of the left atrial appendage LAA first, and then gradually the rest of the compression members contacts the tissue. This provides the squeezing motion away from the left atrium LA that helps prevent extrusion of any thrombus deposits 24 within the cavity 22.

A further alternative method of squeezing the left atrial appendage LAA from the left atrium LA first is to provide multiple compression members on either side of the appendage. Although not shown in the drawings, a first pair of compression members may be deployed close to the exterior wall the left atrium LA and then a second pair deployed in a direction away from the left atrium. This "stepped" compression moving away from the left atrium LA is essentially what occurs when the angled compression members 80a, 80b of Fig. 2E are brought together on either side of the appendage.

Alternatively, or in conjunction with the aforementioned deployment technique, any identified thrombus deposits 24 may be immobilized within the left internal appendage cavity 22 prior to excluding the cavity from the interior of the left atrium LA. For example, a pin or needle may be used to pierce into the identified thrombus deposit 24 from the exterior of the left atrial appendage LAA. The deposit 24 may be identified using ultrasound or other methods.

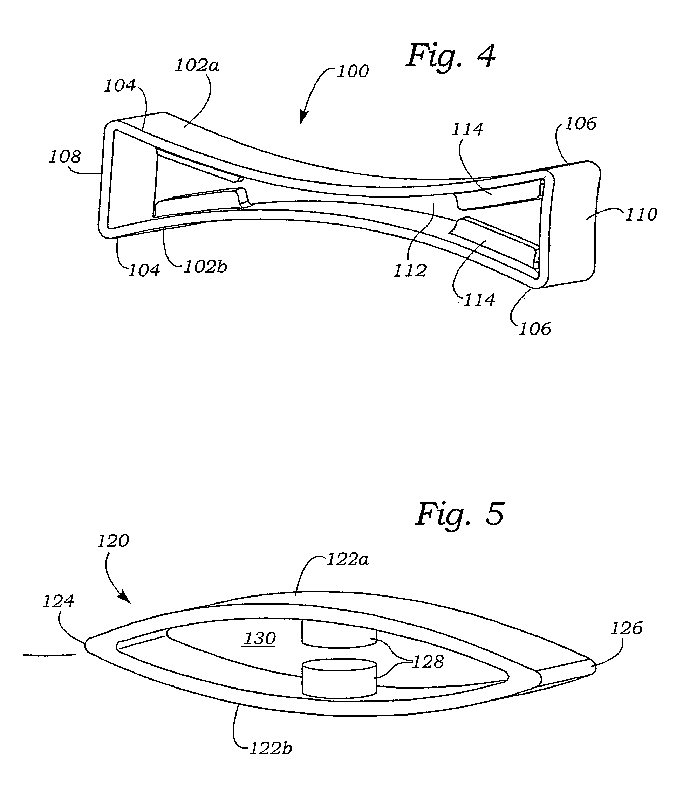

Figs. 4-8 illustrate in perspective several alternative exclusion devices of the present invention. The range of possible configuration should not be considered limited to these exemplary embodiment, though the basic feature of defining a closed periphery with a variable sized opening therein is common to all.

In Fig. 4, a left atrial appendage LAA exclusion device 100 includes upper and lower compression members 102a, 102b having opposed ends 104, 106 that are joined together by hinge walls 108, 110, respectively. The compression members 102a, 102b are shaped similar to the compression members 30a, 30b seen in Figs. 2A-2E, and can be described as elongated, narrow strips. The exclusion device 100 is seen in its relaxed configuration prior to application of any external force, such as during deployment. The relaxed configuration is such that the compression members 102a, 102b lying between the hinge walls 108, 110 are closer together than the ends 104, 106. A closed periphery is therefore defined by the compression members 102a, 102b and hinge walls 108, 110 that is generally rectangular although the compression members arch toward one another (concave to the outside). Because of the elastic properties of the material, such as Delrin or other suitable biocompatible polymer, the compression members 102a, 102b can be forced apart to increase the size of the opening 112 therebetween. The arched shape creates a natural closing bias when the compression members 102a, 102b are separated that is greater in the middle than what would exist if the compression members 102a, 102b were planar in their relaxed configuration.

Each of the compression members 102a, 102b includes a pair of inner protrusions 114 located near the ends 104, 106 and not in the middle. Again, the protrusions 114 are desirably rounded so as to be atraumatic to the left atrial appendage LAA tissue. Although not shown, the protrusions 114 may also include the ramp surface previously described.

Fig. 5 illustrates a still further alternative left atrial appendage LAA exclusion device 120 of the present invention having a lenticular-shaped closed periphery. More specifically, the exclusion device 120 has a pair of spaced apart elongated compression members 122a, 122b joined together at pointed ends 124, 126 and arched convexly away from one another. The resulting shape is lenticular, somewhat like the human eye. Each of the compression members 122a, 122b includes a generally cylindrical protrusion 128 extending inward into the opening 130 defined therebetween. The cylindrical protrusions 128 are located at an approximate midpoint between the ends 124, 126, at the position of greatest separation between the compression members 122a, 122b.

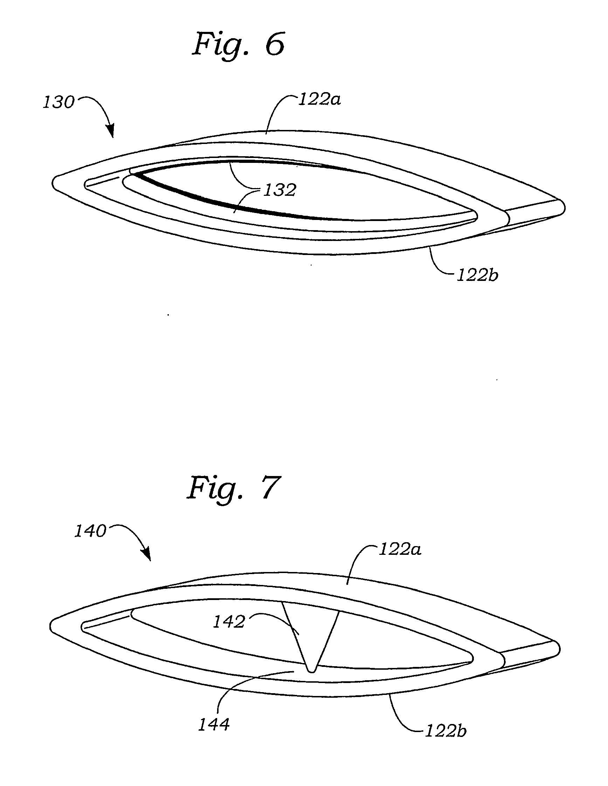

Fig. 6 illustrates another lenticular left atrial appendage LAA exclusion device 130 that is in most respects identical to the device 120 of Fig. 5, except each of the compression members 122a, 122b has an elongated, rounded protrusion or rib 132 extending inward therefrom. Again, this rib 132 helps prevent migration of the device 130 after deployment. The rib may comprise a soft material such as felt and/or a material that may aid in hemostasis.

Fig. 7 shows a still further lenticular left atrial appendage LAA exclusion device 140 having a single protrusion 142 extending inward from only one of the compression members 122a, 122b. The protrusion 142 is conical having a pointed apex 144 intended to more securely anchor the exclusion device to the exterior of the left atrial appendage LAA after deployment.

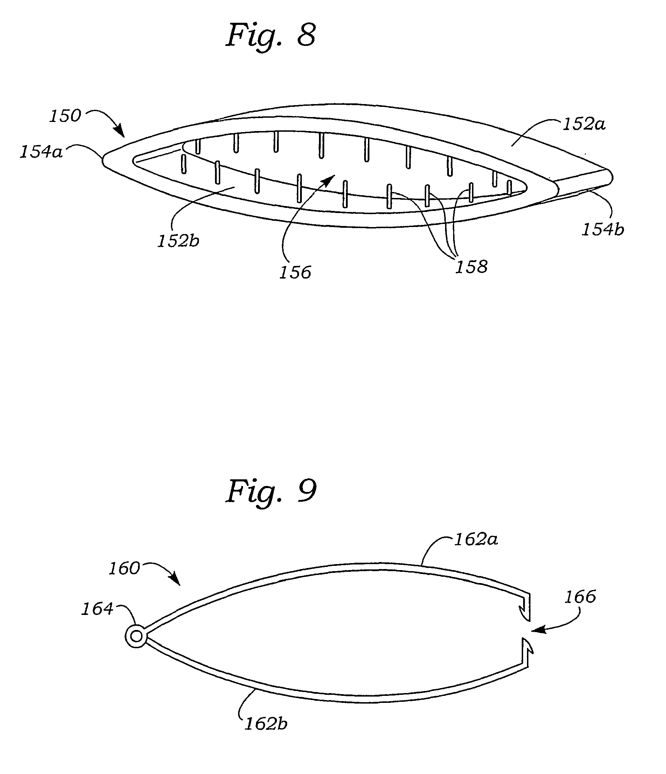

With reference to Fig. 8, an alternative LAA exclusion device 150 of the present invention is shown that has a pair of elongated compression members 152a, 152b joined at opposite ends 154a, 154b. As in the earlier embodiment, the compression members 152a, 152b are concave with respect to one another and together define a lenticular-shaped opening 156. A plurality of piercing members or needles 158 are secured to and extend inward from one or both of the compression members 152a, 152b. The piercing members could be needles or teeth/barbs, and may be straight, angled or slightly curved.

In use, the needles 158 pierce the left atrial appendage tissue and help entrap any pieces of blood clots or other such loose matter within the left atrial appendage cavity. The length of the needles 150a should be sufficient to pass through one wall of the left atrial appendage, and the number of needles should create an array of needles when the device is closed that traps all loose clots or plaque. The clots may be pierced and thus captured directly, or the array of needles may function similarly to the baleen filter of some whales. The needles 158 on the upper compression member 152a are shown offset across the opening 156 from those on the lower compression member 152b, although they could be in alignment. Furthermore, a single row of needles 158 is shown on each compression member 152a, 152b, though it should be understood that multiple rows on each might be provided.

Fig. 9 shows an LAA exclusion device 160 having a pair of separate or discrete compression members 162a, 162b that are hinged together at one end 164 and provided with mating structure 166 on the opposite end. Much like the earlier-described device 20 of Fig. 3, the device 160 is deployed with the mating structure 166 disconnected. The two compression members 162a, 162b are brought together after the device 160 is placed over the left atrial appendage so that the mating structure 166 engages and the compression members define a closed periphery. The hinge 164 may take a variety forms, but is preferably a conventional pin and tube arrangement.

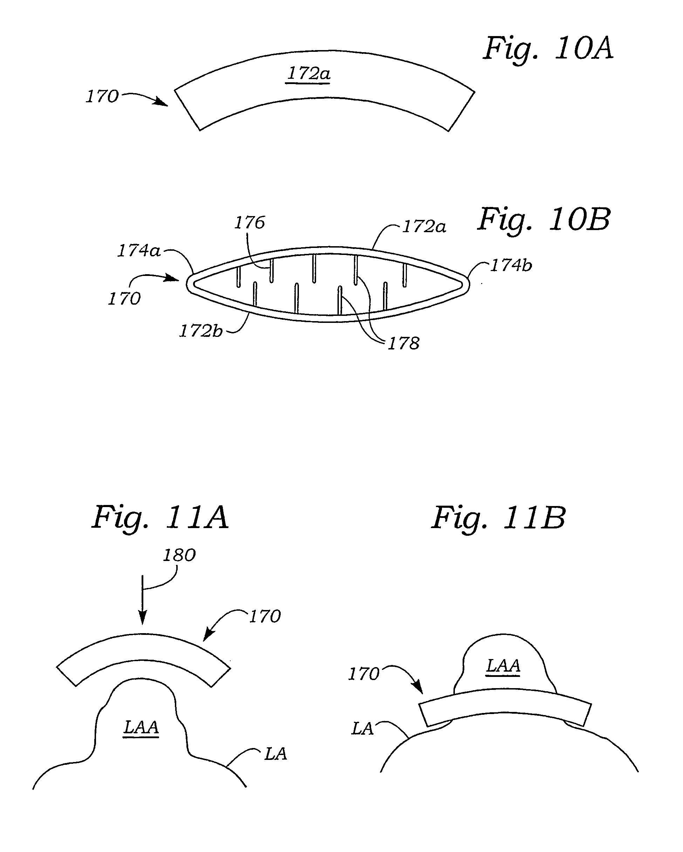

Figs. 10a and 10b are the plan and elevation views of an alternative LAA exclusion device 170 that is in many respects similar to the device 150 shown in Fig. 8. Specifically, the device 170 has a pair of elongated compression members 172a, 172b joined together at opposite ends 174a, 174b and forming a lenticular shaped opening 176. A plurality of needles 178 extend inward from the compression members 172a, 172b for entrapping blood clots within the LAA cavity. As shown in Figure 10a, the compression members 172a, 172b are curved such that the LAA exclusion device 170 has a shape that conforms to the exterior of the left atrium. More generally, the shape of the compression members 172a, 172b is non-linear so as to permit the device 170 to more completely close off the LAA cavity. In the illustrated embodiment, the non-linear shape is an arc that generally conforms to the exterior of the left atrium.

Figs. 11a and 11b illustrate, in plan view, use of the non-linear LAA exclusion device 170 to exclude the left atrial appendage. The device 170 is first spread apart and advanced along arrow 180 toward the LAA. Once positioned adjacent to the exterior wall of the left atrium LA, the device 170 is released and permitted to contract, thus closing off the LAA cavity. As can be seen from Fig. 11b, the non-linear shape of the device 170 closely conforms to the curved exterior of the left atrium LA, and more completely matches the shape of the LAA cavity opening. The compression members 172a, 172b may be formed of a variety of biocompatible materials, and may be molded into the non-linear shape required. Alternatively, the compression members 172a, 172b may be formed of malleable material that can be bent into the desired shape by the surgeon. In the latter case, the device 170 is considered flexible in at least two planes to permit opening and closing, and also to permit shaping of the device to conform to the shape of the left atrium.

Deployment of any of the exclusion devices described herein can be accomplished using an open chest approach, such as a median sternotomy, or with a minimally-invasive approach such as a small thoracotomy or port access procedure. In one exemplary embodiment, deployment will be in a minimally invasive manner, i.e., where access to the pericardial space overlying the patient's left atrial appendage is accomplished through percutaneous penetrations through the patient's skin. Such a technique is disclosed in U.S. Patent No. 6,488,689 to Kaplan, et al. Rather than passing through the rib cage, as with prior thoracoscopic techniques, a "sub-xiphoid" approach is used in Kaplan, et al. where the percutaneous penetration is first made beneath the rib cage, preferably between the xiphoid and adjacent costal cartilage. An LAA exclusion device of the present invention is then advanced through the penetration, over the epicardial surface (in the pericardial space) to reach a location adjacent to the exterior of the left atrial appendage. The exclusion device can then be used to close the left atrial appendage to prevent the formation of clots and the release of emboli from the atrium.

The degree of compression on the tissue of the left atrial appendage is desirably great enough to cause necrosis or fibrosis along a line across the inner cavity mouth. This creates a block of conduction though the tissue which electrically isolates the left atrial appendage and helps reduce problems associated with atrial fibrillation.

Any of the LAA exclusion devices of the present invention may be coated with one or more biocompatible materials to facilitate long-term implantation. For example, the devices may be coated with an agent that is either synthetic or biological, active or inactive. Active agents include heparin and other anticoagulants, EDGF (endothelium derived growth factor), VGFs (vascular growth factors) or inactive agents like silicone, polyurethane, PTFE (polytetrafluoroethylene) and/or other polymers. Inactive (or inert) agents may have active agents such as those listed above encapsulated therewithin. The coating may be bioresorbable or not.

It will also be appreciated by those of skill in the relevant art that various modifications or changes may be made to the examples and embodiments of the invention described in this provisional application, without departing from the intended scope of the invention. In this regard, the particular embodiments of the invention described herein are to be understood as examples of the broader inventive concept disclosed in this application.

## Claims

1. A left atrial appendage (LAA) exclusion device (20), comprising:
a pair of elongated spaced apart compression members (30a, 30b) co-extensive with one another, wherein the members form a closed periphery defining an opening (32) therein when the two ends of the members are brought together; **characterised in that**:
each of the compression members has a ramped protrusion (70a, 70b) forming an angled compression surface, extending along the elongate compression member and facing inward toward the opening (32) and opposite the other protrusion;
**in that** the protrusions co-operate, in use, to provide a compressive force to opposite sides of the left atrial appendage; and
**in that** said angled compression surface (76) provides a squeezing motion, in use, away from the left atrium.

2. The left atrial appendage exclusion device of claim 1 wherein the two members (30a, 30b) are joined together at both ends.

3. The device of claim 1, wherein the compression members (30a, 30b) are flexible and biased toward each other to minimize the opening within the closed periphery.

4. The device of claim 1, wherein the closed periphery defines a lenticular opening.

5. The device of claim 1, wherein the closed periphery defines a generally rectangular opening.

## Patentansprüche

1. Ausschlussvorrichtung (20) für das linke Herzohr (LAA), umfassend:
ein Paar länglicher mit Abstand angeordneter Kompressionselemente (30a, 30b), die miteinander koextensiv sind, wobei die Elemente eine geschlossene Peripherie bilden, die eine Öffnung (32) darin definiert, wenn die zwei Enden der Elemente zusammengebracht werden, **dadurch gekennzeichnet, dass**:
jedes der Kompressionselemente einen rampenförmigen Vorsprung (70a, 70b) aufweist, der eine gewinkelte Kompressionsfläche bildet, die sich entlang des länglichen Kompressionselements erstreckt und in Richtung der Öffnung (32) nach innen gerichtet ist und dem anderen Vorsprung gegenüberliegt;
die Vorsprünge, im Gebrauch, zusammenarbeiten, um entgegengesetzten Seiten des linken Herzohrs eine Druckkraft bereitzustellen; und
besagte gewinkelte Kompressionsfläche (76), im Einsatz, eine Quetschbewegung vom linken Herzvorhof weg bereitstellt.

2. Ausschlussvorrichtung für das linke Herzohr nach Anspruch 1, wobei die zwei Elemente (30a, 30b) an beiden Enden miteinander verbunden sind.

3. Vorrichtung nach Anspruch 1, wobei die Kompressionselemente (30a, 30b) flexibel und gegeneinander vorgespannt sind, um die Öffnung in der geschlossenen Peripherie zu minimieren.

4. Vorrichtung nach Anspruch 1, wobei die geschlossene Peripherie eine linsenförmige Öffnung definiert.

5. Vorrichtung nach Anspruch 1, wobei die geschlossene Peripherie eine generell rechteckige Öffnung definiert.

## Revendications

1. Dispositif d'exclusion (20) d'auricule cardiaque gauche (ACG), comprenant :
une paire de membres de compression allongés et espacés entre eux (30a, 30b), en co-extension l'un par rapport à l'autre, dans lequel les membres forment une périphérie fermée délimitant ainsi en elle une ouverture (32) quand les deux extrémités des membres sont rapprochées entre elles ; **caractérisé en ce que** :
chacun des membres de compression possède une protrusion inclinée (70a, 70b) formant une surface de compression angulaire, s'étendant le long du membre de compression allongé, tournée en dedans et vers l'ouverture (32), et située face à l'autre protrusion ;
**en ce que** les protrusions coopèrent, durant l'utilisation, afin d'infliger une force compressive aux côtés opposés de l'auricule cardiaque gauche ; et
**en ce que** ladite surface de compression angulaire (76) fournit un mouvement exprimeur, durant l'utilisation, à l'écart de l'oreillette gauche.

2. Dispositif d'exclusion d'auricule cardiaque gauche selon la revendication 1 dans lequel les deux membres (30a, 30b) sont fixés entre eux par les deux extrémités.

3. Dispositif selon la revendication 1, dans lequel les membres de compression (30a, 30b) sont flexibles et inclinés l'un vers l'autre afin de minimiser l'ouverture située dans la périphérie fermée.

4. Dispositif selon la revendication 1, dans lequel la périphérie fermée délimite une ouverture lenticulaire.

5. Dispositif selon la revendication 1, dans lequel la périphérie fermée délimite une ouverture généralement rectangulaire.
